# EUROPEAN PATENT APPLICATION

(11) **EP 4 465 041 A1**
(43) Date of publication of application: **20.11.2024**
(21) Application number: 23740260.7
(22) Date of filing: 11.01.2023
(51) Int. Cl.: G01N 33/49, G01N 33/68

(54) **METHOD FOR EXAMINING BLOOD SAMPLE**

(30) Priority: 13.01.2022 JP 2022003487
(71) Applicant: Provigate Inc., Tokyo 113-0033 (JP)
(72) Inventor: NISHI Mitsumi, Tokyo 113-0033 (JP); MIYAUCHI Noriko, Tokyo 113-0033 (JP); IRIE Tomoko, Tokyo 113-0033 (JP)
(74) Representative: Michalski Hüttermann & Partner Patentanwälte mbB
(86) International application number: PCT/JP2023/000380
(87) International publication number: WO 2023/136251

(57) **Abstract**

In an embodiment of the present disclosure, a method for testing a blood sample is provided. The method includes determining whether subject blood is hemolyzed; and, if it is determined that the subject blood is hemolyzed, testing the subject blood with a test instrument that is responsive to hemolysis.

## Description

### TECHNICAL FIELD

The present disclosure relates to a method for testing a blood sample.

### BACKGROUND ART

In blood tests, concentrations of various substances included in blood are measured in various ways. Examples thereof include glycated albumin in blood. Glycated albumin is an advanced glycation product of albumin. Albumin is a protein that is present in abundance in the body and binds to glucose at a high ratio. A degree of glycation (GA value) of glycated albumin is considered to represent blood glucose levels for approximately the most recent two weeks. Accordingly, the GA value has attracted attention in recent years as an index that can be used to control the blood glucose.

Typically, glycated albumin is measured by a high-performance liquid chromatography method (HPLC), an enzymatic method, or the like.

In general, absorbance spectrometers that use an enzymatic method are inexpensive and do not need a large occupancy space. In addition, they allow the use of commercially available reagents and do not require, for example, condition setting or preparation of reagents. In addition, a pretreatment operation is simple (it is sufficient to merely place a blood collection tube directly under centrifugation). However, these are based on an optical measurement, such as an absorbance measurement, and, therefore, in instances where a substance containing a coloring material, such as hemoglobin, is present, that is, in instances where, for example, whole blood is measured, correct measured values cannot be obtained. In other words, in the instance of hemolysis, an accurate measurement cannot be made.

HPLC instruments, in general, can perform a relatively accurate measurement even in the instance of hemolysis. However, they are dedicated instruments and, therefore, expensive, and further, they need a large occupancy space (footprint) and involve a long measurement time.

### SUMMARY OF INVENTION

In instances of blood tests, particularly, in instances where capillary blood is used, hemolysis occurs at a certain frequency, and, therefore, a need exists to address hemolysis. For example and without limitation, recognized herein is a need for a method that enables accurate blood test results to be returned more efficiently, while also addressing the occurrence of hemolysis.

Some embodiments of the present disclosure provide a method for testing a blood sample. In some embodiments, the method includes determining whether subject blood is hemolyzed and, if it is determined that the subject blood is hemolyzed, testing the subject blood with a test instrument that is responsive to hemolysis (hemolysis-responsive test instrument).

Accordingly, when there is no hemolysis, the blood test can be carried out with a non-hemolysis-responsive test instrument, such as an absorbance spectrometer that uses an enzymatic method, and the use of a hemolysis-responsive test instrument, such as an HPLC instrument, for performing the blood test, can be limited to instances in which there is hemolysis. Consequently, for example, the measurement step can be carried out more efficiently for a large number of blood samples, and, therefore, accurate results can be returned. Furthermore, with regard to test laboratories and test spaces having multiple instruments, an efficient arrangement can be made. For example, regarding the respective numbers of non-hemolysis-responsive test instruments and hemolysis-responsive test instruments to be installed in the laboratory and the total number of these, it is possible to select an efficient number for each of them.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the present disclosure. Accordingly, the drawings and description are to be regarded as illustrative in nature, and not as restrictive.

### BRIEF DESCRIPTION OF DRAWINGS

[FIG. 1] FIG. 1 is a graph illustrating a relationship between HPLC and an enzymatic method, regarding a GA% measurement.
[FIG. 2] FIG. 2 is a flowchart illustrating a measurement procedure according to an embodiment.
[FIG. 3] FIG. 3 is a flowchart illustrating a measurement procedure according to an embodiment.
[FIG. 4] FIG. 4 is a flowchart illustrating a measurement procedure according to an embodiment.
[FIG. 5] FIG. 5 is a graph illustrating temporal changes in an enzymatic-method-based absorbance that occurred in instances in which there was hemolysis or in which there was no hemolysis.
[FIG. 6] FIG. 6 is a graph illustrating spectroscopic spectra of an absorbance associated with hemoglobin, according to an example.
[FIG. 7] FIG. 7 is a graph illustrating spectroscopic spectra of an absorbance associated with a protein, according to an example.
[FIG. 8] FIG. 8 is a flowchart illustrating a measurement procedure according to an embodiment.
[FIG. 9] FIG. 9 is a flowchart illustrating a measurement procedure according to an embodiment.
[FIG. 10] FIGS. 10 (A) and (B) are graphs illustrating spectroscopic spectra of an absorbance associated with a protein, according to an example.
[FIG. 11] FIG. 11 is a graph illustrating an effect of a correction of a dilution ratio corresponding to a total protein, according to an example.
[FIG. 12] FIG. 12 is a flowchart illustrating a measurement procedure according to an embodiment.
[FIG. 13] FIG. 13 is a flowchart illustrating a measurement procedure according to an embodiment.
[FIG. 14] FIG. 14 is a schematic diagram illustrating a configuration of a test facility according to an embodiment.

### <1. Difference Between HPLC and Enzymatic Method in GA% Measurement>

Enzymatic methods use an absorbance measurement and are, therefore, likely to be affected by hemolysis. An experiment, described below, was conducted to investigate the influence of hemolysis.

In a measurement performed by HPLC, a Prominence (registered trademark, Shimadzu Corporation) was used. In a measurement performed by an enzymatic method, a Lucica GA-L (registered trademark, Asahi Kasei Pharma Corporation) was used as a reagent, and a DM-JACK Ex+ (registered trademark, Minaris Medical Co., Ltd.) was used as an absorbance spectrometer.

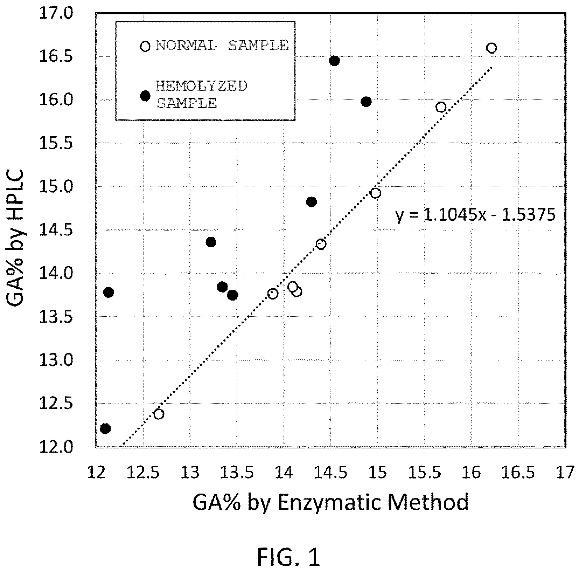

FIG. 1 illustrates a relationship between the HPLC and the enzymatic method, regarding the measured values. The white circle corresponds to a sample having no hemolysis ("normal sample"), and the black circle corresponds to a sample having hemolysis ("hemolyzed sample"). In the normal sample (white circle), GA% values determined by the HPLC method and GA% values determined by the enzymatic method were not significantly different from each other, that is, they were substantially the same values. On the other hand, in the hemolyzed sample, a significant difference was observed between the HPLC method and the enzymatic method. It is known that HPLC methods are not sensitive to hemolysis and, therefore, provide relatively accurate measurement results. On the other hand, enzymatic methods are sensitive to hemolysis. In the hemolyzed sample (black circle) that was hemolyzed, it is apparent that the enzymatic method provided a GA% lower than the correct value (provided by the HPLC method). Accordingly, it was found that a very effective way for carrying out the measurement is a way in which a determination is made as to whether or not a blood sample is hemolyzed, and then, if the blood sample is hemolyzed, a hemolysis-responsive test instrument, such as an HPLC, is used.

### <2. Measurement Method>

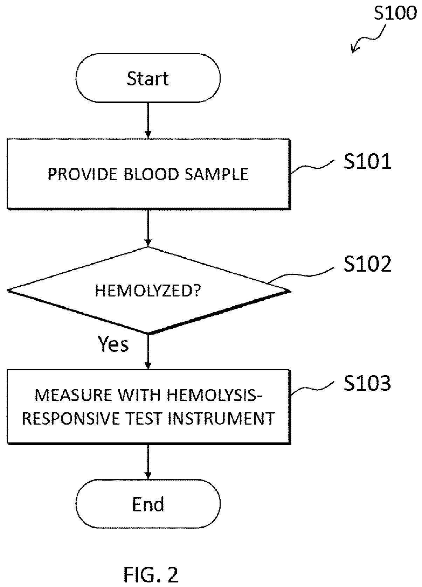

FIG. 2 is a flowchart S 100, which illustrates a test method according to an embodiment.

A blood sample is provided (S101).

Next, a determination is made as to whether the blood sample is hemolyzed (S102).

If the blood sample is hemolyzed, a measurement is performed with a hemolysis-responsive test instrument (S 103).

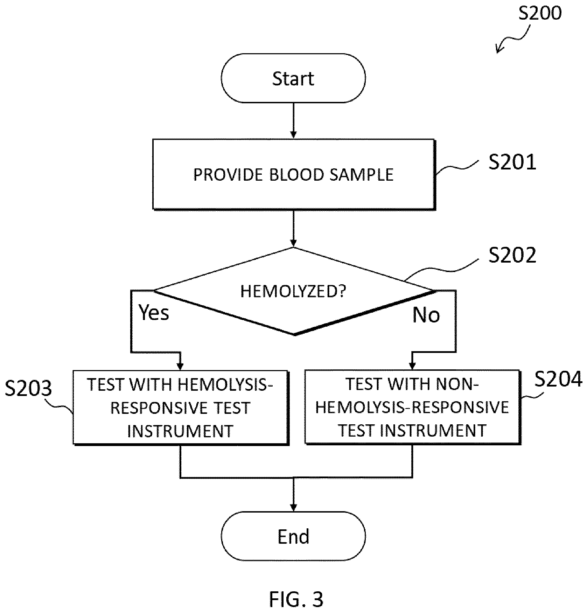

If the blood sample is not hemolyzed, the measurement may be performed with a non-hemolysis-responsive test instrument. FIG. 3 is a flowchart S200, which illustrates a test method according to such an embodiment.

A blood sample is provided (S201).

Next, a determination is made as to whether the blood sample is hemolyzed (S202).

If the blood sample is hemolyzed, a measurement is performed with a hemolysis-responsive test instrument (S203).

If the blood sample is not hemolyzed, a measurement is performed with a non-hemolysis-responsive test instrument (S204).

The blood sample can be provided in various ways. The blood sample may be taken from the vein or artery of a living organism. The blood sample may be taken from droplets (capillary blood) that are generated on the skin by puncturing a fingertip, an earlobe, or the like. In some embodiments, the blood sample may be taken in the vicinity of the test instruments (e.g., in a clinic where the test instruments are installed).

In some embodiments, the blood sample may be taken in a location distant from the test instruments and then transported to a location in which the test instruments are installed. The transport may be carried out via mail or by a carrier. A temperature during the transport may be controlled, and the temperature may be measured or estimated. For example, the blood sample may be maintained at ambient temperature, refrigerated, or frozen, during the transport. Before being transported, the blood sample may be diluted with a predetermined diluent.

The determination regarding hemolysis may be made at any of various times, for example, at the time of the drawing of blood, before mailing, or at the time of reception at a test facility. If there is hemolysis at the time of the drawing of blood, for example, it may be determined, at that time, that a hemolysis-responsive test instrument is to be used for the measurement. If there is no hemolysis at the time of the drawing of blood, for example, it may be determined, at that time, that a non-hemolysis-responsive test instrument is to be used, or that the determination regarding hemolysis is to be made again before the measurement.

As used in this specification, the terms "hemolysis-responsive test" and "hemolysis-responsive test instrument" refer to a measurement that can provide desired results even when there is hemolysis and to an instrument for the measurement. In some embodiments, the hemolysis-responsive test instrument may be an HPLC instrument. The hemolysis-responsive test instrument is not limited to this. The hemolysis-responsive test instrument may be a mass spectrometer (MS). The hemolysis-responsive test instrument may be an instrument that uses an antigen antibody reaction, the boronic acid affinity principle, or the like.

As used in this specification, the terms "non-hemolysis-responsive test" and "non-hemolysis-responsive test instrument" refer to a measurement that cannot provide desired results when there is hemolysis and to an instrument for the measurement. In some embodiments, the non-hemolysis-responsive test instrument may be an instrument that uses an enzymatic method. The non-hemolysis-responsive test instrument is not limited to this. The non-hemolysis-responsive test instrument may be an instrument that uses an antigen antibody reaction, the boronic acid affinity principle, or the like.

In some embodiments, the non-hemolysis-responsive test instrument may be an instrument that performs an absorbance measurement. In some embodiments, the determination as to whether the subject blood is hemolyzed may be made based on the difference in the absorbance between the subject blood and a control sample.

The testing of subject blood may include diabetes tests. One of, at least one of, or both of the hemolysis-responsive test instrument and the non-hemolysis-responsive test instrument may be a diabetes test instrument.

In some embodiments, the testing of subject blood may be a measurement of the GA% (GA value) or may include the measurement. The ratio (GA value or GA%) may be determined, for example, from a total albumin (ALB) concentration and a glycated albumin (GA) concentration.

In the blood test, one or more other parameters may be alternatively or additionally used.

For example, an albumin concentration, a glycated albumin concentration, a glucose concentration, a 1,5-AG concentration, an oxidized albumin concentration, and an oxidized protein concentration may be measured.

Other non-limiting examples of items that may be measured include the following:
biochemical test-related substances, such as total protein (TP), albumin (ALB), oxidized albumin, AST, ALT, γ-glutamyl transpeptidase (γ-GT), urea nitrogen (BUN), creatinine (CRE), creatine kinase (CK), CK-MB activity, amylase (AMY), pancreatic lipase (LIP), uric acid (UA), total cholesterol (T-CHO), neutral fat (TG), HDL cholesterol (HDL-C), LDL cholesterol (LDL-C), iron (Fe), and zinc (Zn);
vitamin-related substances;
electrolyte/blood gas-related substances, such as calcium, potassium, and magnesium;
biochrome-related substances; such as total bilirubin;
occupational toxic metabolites;
antibiotics, antiepileptics, immunosuppressants, cardiovascular drugs, neuropsychiatric drugs, and other drugs;
coagulation and fibrinolysis test-related substances, such as fibrinogen (FIB) and D-dimer;
hormones and binding proteins, such as thyroid stimulating hormone (TSH), adrenocorticotropic hormone (ACTH), cortisol, adrenal medullary hormone, gonadal hormone, and insulin;
endocrinology-related substances, such as human brain natriuretic peptide (BNP) and human N-terminal pro-brain natriuretic peptide (NT-proBNP);
tumor marker-related substances, such as prostate-specific antigen (PSA);
inflammation-related substances, such as CRP;
hepatitis viruses (type B and type C), human immunodeficiency viruses (HIV), and other various viruses;
infectious disease-related substances, such as candida antibodies;
autoimmunity test-related substances;
immunohematology test-related substances;
various immunoglobulins;
various cytokines;
allergy test-related substances;
various amino acids; and
autoantibody test-related substances.

### <3. Determination Regarding Hemolysis>

### <Determination 1 Regarding Hemolysis: Absorbance Measurement That Uses Enzymatic Method>

In some embodiments, the determination regarding hemolysis may be made by using an absorbance measurement. For example, an enzymatic method may be used.

In many cases, absorbance measurements that use an enzymatic method are sensitive to hemolysis. When there is hemolysis, the method can indicate the GA value as a falsely low value. In instances where an anomaly is found in the measured value obtained in the method, a determination may be made that the method is not to be used for the GA value measurement, and that, instead, an HPLC method, which is not sensitive to hemolysis, is to be used for the GA value measurement.

### <Determination 2 Regarding Hemolysis: Measurement That Uses Non-Hemolysis-Responsive Test Instrument>

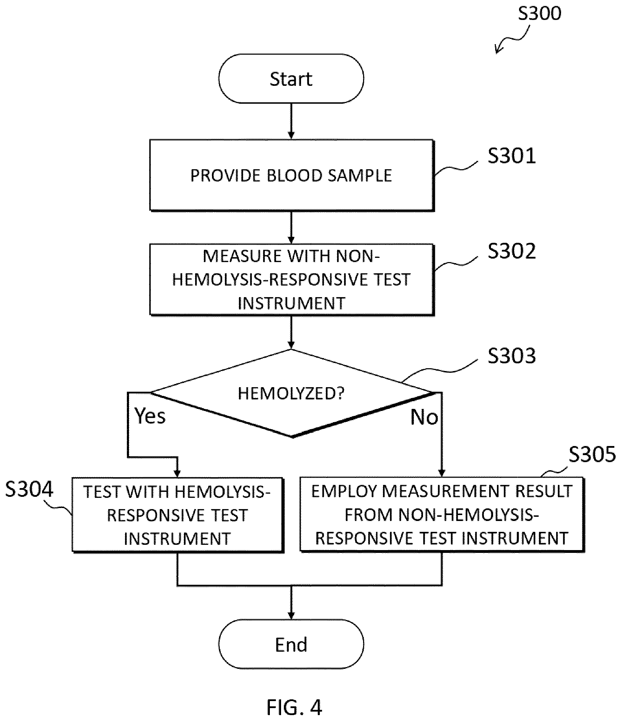

The determination regarding hemolysis may be made by using a non-hemolysis-responsive test instrument. FIG. 4 is a flowchart S300, which illustrates a test method according to such an embodiment.

A blood sample is provided (S301).

A measurement is performed with a non-hemolysis-responsive test instrument (S302).

Based on the measurement, a determination is made as to whether the blood sample is hemolyzed (S303).

If the blood sample is hemolyzed, a measurement is performed with a hemolysis-responsive test instrument (S304).

If the blood sample is not hemolyzed, a measurement is performed with a non-hemolysis-responsive test instrument (S305).

In some embodiments, the determination regarding hemolysis made by using a non-hemolysis-responsive test instrument may be carried out by performing a measurement associated with the target test and using the measurement data. In some embodiments, a predetermined measurement for the determination regarding hemolysis, which is different from the measurement associated with the target test performed by a non-hemolysis-responsive test instrument, may be performed.

### <Example 1>

This Example used a DM-JACK Ex+ (Minaris Medical Co., Ltd.) as an absorbance spectrometer. A standard curve was pre-generated with a Lucica GA-L calibrator (Asahi Kasei Pharma Corporation). The assay reagents used were Lucica GA-L GA R1, R2 and Lucica GA-L ALB R1, R2 (Asahi Kasei Pharma Corporation).

Six blood samples were prepared. First, the whole blood of these was centrifuged to separate the serum from the blood cells. The serum samples were each placed in a sample cup and mounted to a rack for a GA% measurement. Once the measurements were started, the instrument automatically performed the measurements of the GA concentration and the ALB concentration and returned the result value of the GA%.

In the case of the measurement of the GA concentration with the Lucica reagent, a specified dominant wavelength is 546 nm, and a specified complementary wavelength is 700 nm. Hemoglobin has absorption wavelengths at 414 nm, 541 nm, and 576 nm. Accordingly, hemolysis shifts the absorbance at 546 nm in a positive direction.

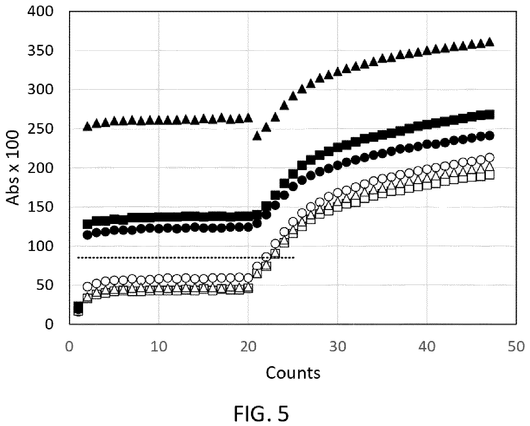

FIG. 5 illustrates temporal changes in the measured absorbances of the six blood samples. The horizontal axis represents counts (one count corresponds to approximately 12 seconds), and the vertical axis represents the absorbance×100. The Lucica reagent R1 was added to the measurement cell at count 1, the serum sample at count 2, and the resultant was heated with stirring continuously. The reagent R2 was added at count 20. The absorbance was measured at each count.

The presence or absence of hemolysis was determined based on the absorbances obtained over the period from the time at which the reagent R1 was added to the time at which the reagent R2 was added.

As is apparent from FIG. 5, the samples that were hemolyzed had higher absorbances than the samples that were not hemolyzed. Referring to FIG. 5, a concentration range that could affect hemolysis was preliminarily determined by using an interference checker, and based on the result, a threshold value of 0.85 (Abs×100=85) was set (dotted line). The presence or absence of hemolysis can be determined based on a relationship between the threshold value and the absorbance determined by the measurement. Samples (outlined white symbols) that exhibited a value smaller than the threshold value can be determined not to be hemolyzed, and samples (black solid symbols) that exhibited a value greater than the threshold value can be determined to be hemolyzed. The threshold value is merely an example. The threshold value may be set in accordance with, for example, the measurement conditions or attributes or properties of the blood, before, during, or after the measurement.

On the other hand, the GA concentration is determined from the difference between an absorbance A1, which is obtained at count 45, and an absorbance A0, which is obtained at count 19, by using a standard curve.

The absorbance A0, which is obtained before coloration (addition of the reagent R2), is strongly affected by hemolysis and increases as a result of hemolysis. On the other hand, the absorbance A1, which is obtained after coloration (addition of the reagent R2), is unlikely to be affected by hemolysis or increased by hemolysis, because the absorbance due to the GA concentration is inherently high. That is, when there is hemolysis, the difference between the absorbance A0 and the absorbance A1 is reduced, and, consequently, the GA concentration is indicated as a falsely low value.

In contrast, in the case of the measurement of the ALB concentration, a specified dominant wavelength is 600 nm, and a specified complementary wavelength is 660 nm. Accordingly, the measured value of the ALB concentration is less likely to be affected by hemolysis than that of the GA concentration. Thus, since the GA% is the result of dividing the GA concentration by the ALB concentration, the GA% is indicated as a falsely low value as a result of hemolysis.

This is the reason that the values of the hemolyzed samples (black solid symbols) obtained in the absorbance measurement with the enzymatic method were smaller, as illustrated in FIG. 1, than the values obtained by HPLC, which were relatively correct results.

<Determination 3 Regarding Hemolysis: Measurement That Uses Microspectrophotometer>

In some embodiments, the determination regarding hemolysis may be made by using a microspectrophotometer.

### <Example 2>

A portion of the subject blood sample that was obtained may be taken, and the absorbance measurement may be performed on the portion. For example, a portion (approximately 2 µL) of a fingertip blood sample (approximately 10 µL) may be taken. The fingertip blood sample (approximately 10 µL) may be diluted 10-fold, and a portion (approximately 2 µL) of the 100 µL of the sample may be taken, for example. The determination regarding hemolysis may be performed on the portion (approximately 2 µL) by using a microspectrophotometer. A correlation between the absorbance and a degree of hemolysis may be preliminarily investigated. The degree of hemolysis may be estimated from the absorbance determined by the measurement.

This Example used a DS-11 (DeNovix Inc.) as a microspectrophotometer. Whole blood (10 µL) was diluted with a buffer solution (90 µL) to a concentration suitable for a measurement, and 2 µL of a supernatant from centrifugation was subjected to the measurement that used the spectrophotometer. An optical absorption spectrum was measured over a range of 190 nm to 840 nm. Consequently, the determination regarding hemolysis was made by examining the absorbance at approximately 414 nm.

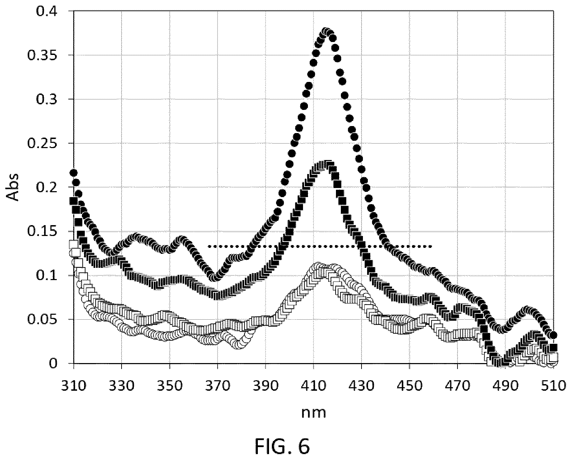

FIG. 6 illustrates absorbance spectra of four serum samples in the vicinity of 414 nm, which is one of the absorption wavelengths of hemoglobin. Referring to FIG. 6, a specified threshold value is 0.13. Samples that exhibited an absorbance less than the threshold value at 414 nm are determined not to be hemolyzed, and samples that exhibited an absorbance greater than the threshold value are determined to be hemolyzed.

### <Others>

The determination regarding hemolysis may be made in a different method or manner, without being limited to the above-described embodiments or examples. For example, the determination may be made by visual inspection. For example, a blood sample taken from the vein may be visually inspected to determine the presence or absence of hemolysis. For example, after centrifugation, whether the supernatant is hemolyzed may be determined by using a background of white light. For example, colorimetry may be used for the determination. A hemoglobin concentration of the subject blood may be measured with a hemoglobin concentration assay kit. A different method may be used. For example, the determination may be made by using an immunoturbidimetric method, an optical reflectance, a near-infrared spectroscopic imaging method, an HPLC method, or the like.

### <4. Determination Based on Protein Content and Adjustment of Sample>

When the concentration of a protein in the blood sample is lower than a specified value, or when an amount of blood drawn is insufficient, an error in the HPLC measurement can increase. In such instances, by reducing a dilution ratio (increasing the concentration of proteins), the protein concentration can be adjusted to an appropriate value, or the amount of the measurement sample can be increased. Note that regarding the HPLC measurement, the measurement can be carried out as long as an amount of proteins is not extremely small.

In some embodiments, when a total concentration of proteins is low, an error in the measurement performed by a non-hemolysis-responsive test instrument (e.g., an absorbance measurement that uses an enzymatic method) increases. In addition, when the dilution ratio is too low, the total amount necessary for the measurement performed by a non-hemolysis-responsive test instrument cannot be ensured. Accordingly, in such instances, a determination may be made that the measurement is to be performed by an HPLC method (i.e., by a hemolysis-responsive test instrument).

On the other hand, when the concentration of proteins in the blood sample is higher than a specified value, the dilution ratio may be increased. Alternatively, the amount of introduction (injection) into the HPLC may be reduced. Consequently, the number of times that the measurement is performed increases, and, therefore, the blood sample provided can be effectively used. Furthermore, a load on the column of the HPLC can be reduced. In some embodiments, the concentration of proteins may be measured with a microspectrophotometer (e.g., NanoDrop (registered trademark)). This measurement may be performed together with the determination regarding hemolysis.

### <Example 3>

This Example used a DS-11 (DeNovix Inc.) as a microspectrophotometer. The procedure for the experiment was similar to that of Example 2.

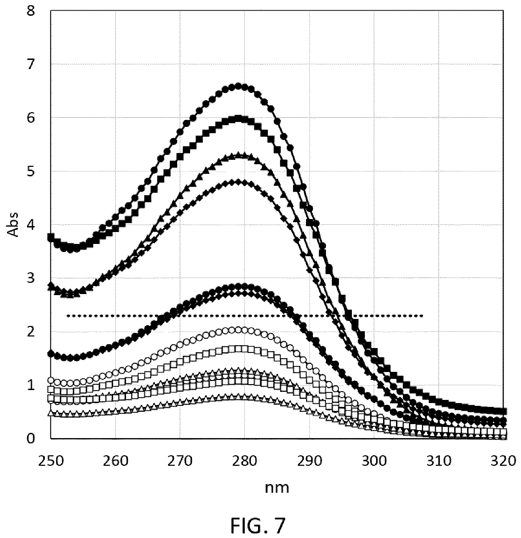

FIG. 7 illustrates absorbance spectra of twelve serum samples in the vicinity of 280 nm. Referring to FIG. 7, a specified threshold value is 2.2. Regarding whether a total amount of proteins in each of the samples is high or low, a determination can be made based on the threshold value.

### <Selection of Measurement Instrument Based on Determination Regarding Hemolysis and on Determination of Total Amount of Proteins>

The measurement instrument to be used may be selected based on both the determination regarding hemolysis and the determination of the total amount of proteins. In some embodiments, when there is no hemolysis, and the total amount of proteins is not excessively low (or is appropriate), the measurement may be performed with a non-hemolysis-responsive test instrument.

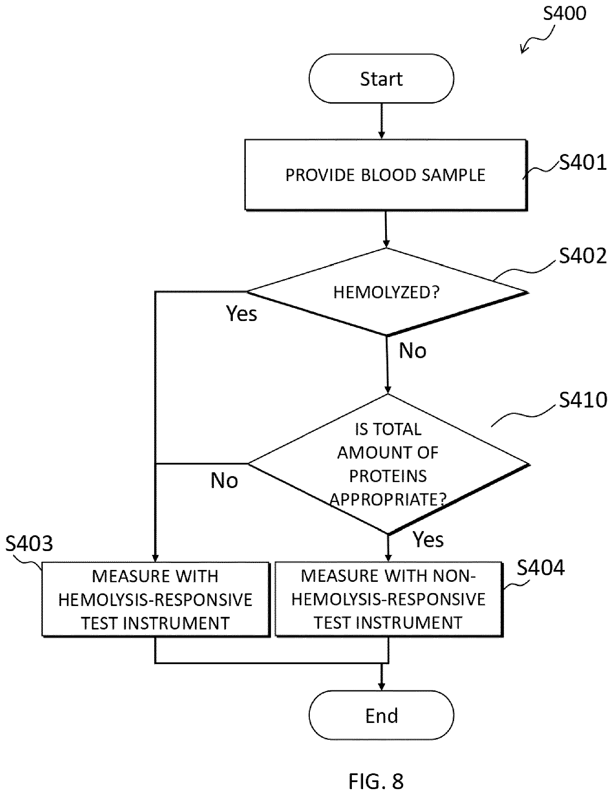

FIG. 8 is a flowchart S400, which illustrates a test method according to such an embodiment.

A blood sample is provided (S401).

A determination is made as to whether the blood sample is hemolyzed (S402).

If the blood sample is hemolyzed, a measurement is performed with a hemolysis-responsive test instrument (S403).

If the blood sample is not hemolyzed, a determination is made as to whether the total amount of proteins is within an appropriate range (S410).

If the total amount of proteins is not within the appropriate range, the measurement is performed with a hemolysis-responsive test instrument (S403).

If the total amount of proteins is within the appropriate range, the measurement is performed with a non-hemolysis-responsive test instrument (S404).

In some embodiments, the determination regarding hemolysis (S402) and the determination of the total amount of proteins (S410) may be made in the opposite order.

### <5. Selection of Conditions for Hemolysis-Responsive Test Instrument Based on Determination of Total Amount of Proteins>

In some embodiments, conditions of use (e.g., measurement conditions) of the hemolysis-responsive test instrument may be selected based on the determination of the total amount of proteins.

In some embodiments, the sample dilution ratio may be modified or adjusted based on the determination of the total amount of proteins. If the amount of blood drawn is less than a specified amount, the blood test cannot be performed appropriately in some cases. For example, in the case of the test involving transportation, the state of the blood drawn depends on the subject, and, therefore, the amount of blood drawn is difficult to ascertain. Furthermore, in the case of the test involving transportation, the whole blood may be one that was diluted after the blood had been drawn. In such cases, it is difficult to calculate, after the transportation and reception, the original amount of blood drawn, based on the amount of the sample containing the diluent. On the other hand, the total amount of proteins, in many cases, is relatively stable for each subject. Accordingly, the amount of blood drawn can be estimated from the total amount of proteins present in the received blood sample.

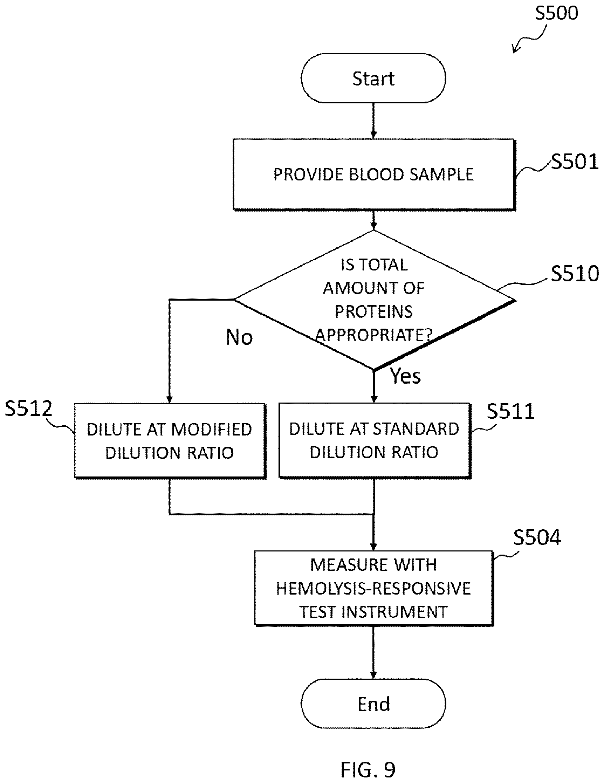

FIG. 9 is a flowchart S500, which illustrates a test method according to such an embodiment.

A blood sample is provided (S501).

A determination is made as to whether the total amount of proteins present in the provided blood sample is within an appropriate range (S510).

If the total amount of proteins is within the appropriate range, the blood sample is diluted at a standard dilution ratio (S511).

If the total amount of proteins is not within the appropriate range, the blood sample is diluted at a dilution ratio that has been modified from the standard dilution ratio (S512).

After being diluted at an appropriate dilution ratio (S511 or S512), the blood sample is measured with a hemolysis-responsive test instrument (S504).

The appropriate range of the total amount of proteins (S510) may be defined by a lower-limit threshold value. If the total amount of proteins is less than the lower-limit threshold value, the amount of blood drawn can be determined to be small. The appropriate range of the total amount of proteins (S510) may be defined by an upper-limit threshold value. If the total amount of proteins is greater than the upper-limit threshold value, the amount of blood drawn can be determined to be large. In addition, a constitution, a physical condition, a health status, or a medical status of a subject, or a change therein may be determined.

### <Example 5>

If the total amount of proteins is not within an appropriate range, even a hemolysis-responsive test instrument, such as an HPLC, can provide a false measurement result.

This Example used blood samples from two subjects, one of whom had a GA% of 12, and the other of whom had a GA% of 16. Four samples having respective volumes of 5 µL, 8 µL, 10 µL, and 15 µL were prepared from each of the blood samples, that is, eight samples in total were prepared. The blood samples in the amounts (5 µL and 8 µL) smaller than a specified value and the blood sample in the amount (15 µL) greater than the specified value were prepared for a simulation. The total amount of proteins in the samples prepared as described was evaluated with a DS-11 (DeNovix Inc.), which is a microspectrophotometer.

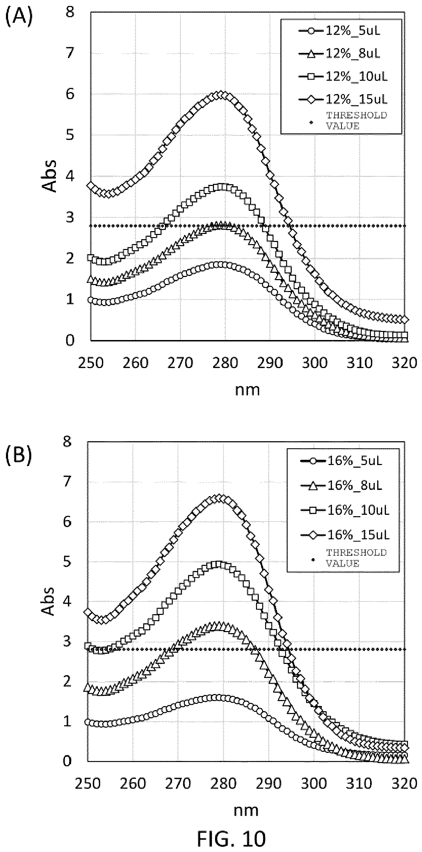

FIG. 10 illustrates absorbance spectra in the vicinity of 280 nm. The illustrated absorbance spectra were those of four samples obtained from each of the blood samples of the subjects, one of whom had a GA% of 12 (FIG. 10A), and the other of whom had a GA% of 16 (FIG. 10B). A specified threshold value at 280 nm, at which a maximum peak is observed, is 2.8.

In both cases, the 8-µL, 10-µL, and 15-µL samples exhibited an absorbance higher than the threshold value and, therefore, can be diluted at a standard dilution ratio. In contrast, the 5-µL samples exhibited an absorbance lower than the threshold value, in both cases. This result indicates that the dilution ratio needs to be modified.

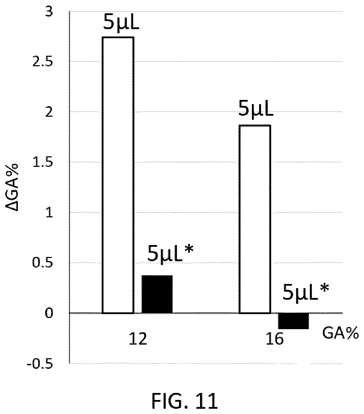

An actual measurement performed by an HPLC shows that the GA% of the 5-µL sample is higher than that of the sample having the specified amount of 10 µL. FIG. 11 illustrates ΔGA% = [GA% in the case of 5 µL] - [GA% in the case of the specified amount of 10 µL] (outlined white bar graph, denoted as "5 µL"). The ΔGA% of the subject with a GA of 12% was approximately 2.75%. The ΔGA% of the subject with a GA of 16% was approximately 1.8%.

Accordingly, in either case, an accurate value cannot be obtained if the dilution is performed at a standard dilution ratio. Accordingly, an appropriate dilution ratio may be determined by estimating the actual amount of blood drawn, based on the absorbance at 280 nm, illustrated in FIG. 10. Dilution was performed at a modified dilution ratio, and then, the GA% was measured again with the HPLC. The ΔGA% is illustrated in FIG. 11 (black solid bar graph, denoted as "5 µL*"). The ΔGA% of the subject with a GA of 12% was approximately 0.3%. The ΔGA% of the subject with a GA of 16% was approximately - 0.2%. That is, in both cases, an accurate GA% could be obtained by employing an appropriate dilution ratio.

In some embodiments, the determination regarding hemolysis may not be necessarily performed. In some embodiments, a facility for the blood test need not be equipped with an instrument for the determination regarding hemolysis and/or with a non-hemolysis-responsive test instrument.

### <5. Determination Based on Amount of Sample and Adjustment of Sample>

In some embodiments, the measurement instrument to be used may be selected, and/or the dilution ratio may be modified, based on the amount of the blood sample provided. For example, if the amount of the blood sample provided is less than a specified amount, it may be determined that a non-hemolysis-responsive test instrument is not to be used, and/or that a hemolysis-responsive test instrument is to be used. For example, if the amount of the blood sample provided is less than a specified amount, a dilution ratio that is different from a standard dilution ratio and suited to the use conditions of the hemolysis-responsive test instrument may be employed.

In some embodiments, a determination as to which of a hemolysis-responsive test instrument and a non-hemolysis-responsive test instrument is to be used may be made based on an amount of the sample and on the presence or absence of hemolysis, and in addition, the sample dilution ratio for a hemolysis-responsive test instrument may be determined based on the total amount of proteins.

The determination of the amount of the sample may be made at any of various times, for example, at the time of the drawing of blood, before mailing, or at the time of reception at a test facility. The amount of the sample may be determined, for example, by taking a picture of a blood collection tube or the like containing the blood sample and using the image. For example, when a data representation (e.g., a bar code or a QR code (registered trademark)) attached to or accompanying a blood collection tube or a container for transportation is photographed, an image of the interior, associated with the amount of the sample, may be acquired.

If an amount, such as the amount of the blood sample or the amount of blood cell components, does not satisfy a predetermined amount, it may be determined that the amount of blood drawn was insufficient and/or that, in the instance where dilution is performed before the transportation of the drawn blood, the amount of the diluent was small.

If an amount, such as the amount of the blood sample or the amount of blood cell components, does not satisfy a predetermined amount, for example, if the amount of the whole sample is smaller than an expected amount or is obviously smaller (the amount of the sample is less than the amount of blood drawn), some possibilities may be considered, which include a possibility that the container for the drawn blood provided to a subject originally contained no diluent, a possibility that the amount of the diluent that was contained was less than a specified amount, and a possibility that a diluent was spilled during use.

Subsequently, any of the following may be performed: to instruct the subject to have blood drawn again, to instruct the subject to note that the drawing of blood in the current instance may not necessarily result in the return of correct results, and to instruct a person in charge of the measurement to modify the conditions for the test or the measurement or the way that the measurement data is handled.

In some embodiments, the amount of the blood sample may be an amount of blood cell components. For example, a volume of red blood cell components resulting from natural settling, centrifugation, or the like may be designated as the amount of the blood sample. The amount of blood drawn may be determined from the amount of the blood sample.

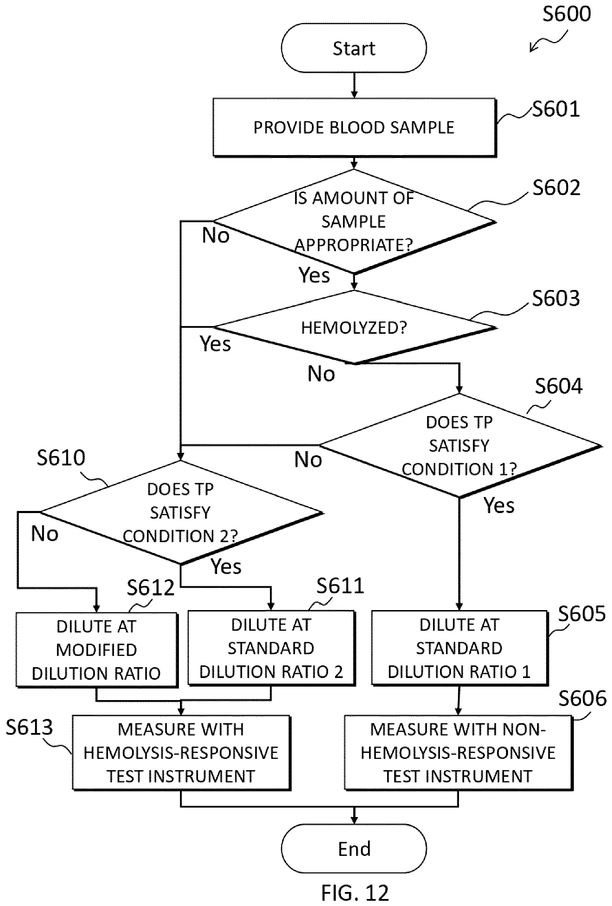

FIG. 12 is a flowchart S600, which illustrates a test method according to such an embodiment.

A blood sample is provided (S601).

A determination is made as to whether the amount of the blood sample provided is appropriate for a non-hemolysis-responsive test instrument (S602). If the amount of the sample is not appropriate, it is determined that a hemolysis-responsive test instrument is to be used.

If the amount of the sample is appropriate, a determination is made as to whether the blood sample is hemolyzed (S603). If the blood sample is hemolyzed, it is determined that a hemolysis-responsive test instrument is to be used.

If the blood sample is not hemolyzed, a determination is made as to whether the total amount of proteins (TP) is appropriate for a non-hemolysis-responsive test instrument (whether condition 1 is satisfied) (S604). If the total amount of proteins is not appropriate for a non-hemolysis-responsive test instrument (condition 1 is not satisfied), it is determined that a hemolysis-responsive test instrument is to be used.

If the total amount of proteins (TP) is appropriate for a non-hemolysis-responsive test instrument (condition 1 is satisfied), the sample is diluted at a standard dilution ratio for a non-hemolysis-responsive test instrument (standard dilution ratio 1) (S605).

The blood sample diluted at the standard dilution ratio 1 is measured with a non-hemolysis-responsive test instrument (S606).

If, at steps S602, S603, and/or S604, it is determined that a hemolysis-responsive test instrument is to be used, a determination is made as to whether the total amount of proteins (TP) is appropriate for a standard measurement performed by a hemolysis-responsive test instrument (condition 2 is satisfied) (S610).

If the total amount of proteins is appropriate for the standard measurement performed by a hemolysis-responsive test instrument (condition 2 is satisfied), the sample is diluted at a standard dilution ratio for a hemolysis-responsive test instrument (standard dilution ratio 2) (S611).

If the total amount of proteins is not appropriate for the standard measurement performed by a hemolysis-responsive test instrument (condition 2 is not satisfied), the blood sample is diluted at a dilution ratio modified from the standard dilution ratio (S612).

The sample diluted at the standard dilution ratio 2 (S611) or the sample diluted at the modified dilution ratio (S612) are measured with a hemolysis-responsive test instrument (S613).

In the above embodiment, the measurement of the total amount of proteins is described as two steps (S604 and S610), corresponding respectively to the presence and absence of hemolysis; however, the measurement is not limited to this. For example, the measurement of the total amount of proteins may be performed in a single step, regardless of the presence or absence of hemolysis. Two specified values TP1 and TP2 (>TP1) of the total amount of proteins may be set, for example. If the total amount of proteins TP is equal to or greater than the higher value TP1 (TP1 </≤ TP), dilution may be performed at the standard dilution ratio 1, and the measurement may be performed with a non-hemolysis-responsive test instrument. If the total amount of proteins TP is between the higher value TP1 and the lower value TP2 (TP2 </≤ TP </≤ TP1), for example, dilution may be performed at the standard dilution ratio 2, and the measurement may be performed with a hemolysis-responsive test instrument. If the total amount of proteins TP is equal to or less than the lower value TP2 (TP </≤ TP2), for example, dilution may be performed at a lower dilution ratio (modified dilution ratio), and the measurement may be performed with a hemolysis-responsive test instrument.

In some embodiments, the blood sample may be centrifuged. The amount of the sample may be determined based on the amount of the blood cell components resulting from the centrifugation. In some embodiments, the blood sample need not be diluted for the measurement performed by a non-hemolysis-responsive test instrument, that is, the blood sample provided may be directly used.

### <6. Determination Based on Turbidity and Adjustment of Sample>

In some cases, when a concentration of lipid in the blood sample is higher than a specified value, the measurement cannot be performed with a non-hemolysis-responsive test instrument. In some other cases, when the concentration of lipid in the blood sample is even higher, the HPLC measurement cannot be performed appropriately. If a turbidity mainly due to lipid is high, it is necessary to determine that dilution is to be performed or that the measurement is not to be performed.

In some embodiments, the measurement instrument to be used may be selected, the dilution ratio may be modified, and/or the determination as to whether or not the measurement is possible may be made based on the turbidity. For example, if the turbidity is less than a specified amount, it may be determined that the use of any non-hemolysis-responsive test instrument is to be avoided, and/or that a hemolysis-responsive test instrument is to be used. For example, if the amount of the blood sample provided is less than a specified amount, a dilution ratio that is different from the standard dilution ratio and suited to the conditions of use of a hemolysis-responsive test instrument may be employed.

In some embodiments, a determination as to which of a hemolysis-responsive test instrument and a non-hemolysis-responsive test instrument is to be used may be made based on the amount of the sample, the presence or absence of hemolysis, and the turbidity, and the sample dilution ratio for the hemolysis-responsive test instrument may be determined, or a determination that the measurement is not possible may be made, based on the total amount of proteins and on the turbidity.

The measurement of the turbidity and a determination based on the measurement may be made at any of various times, for example, at the time of the drawing of blood, before mailing, or at the time of reception at a test facility. Visual inspection, for example, may be performed. An absorbance measurement, for example, may be performed.

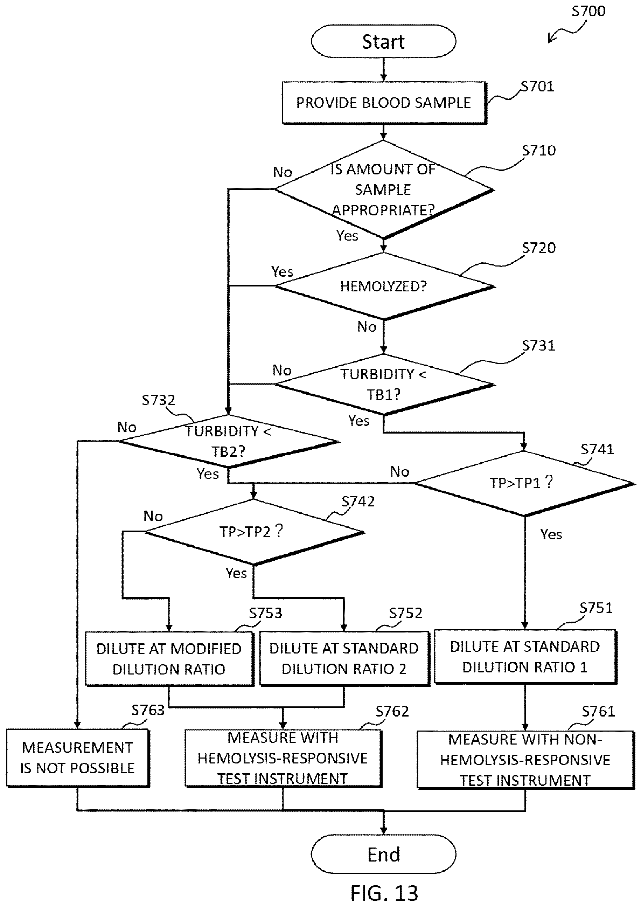

FIG. 13 is a flowchart S700, which illustrates a test method according to such an embodiment.

A blood sample is provided (S701).

A determination is made as to whether the amount of the blood sample provided is appropriate for a non-hemolysis-responsive test instrument (S710). If the amount of the sample is not appropriate, it is determined that a hemolysis-responsive test instrument is to be used.

If the amount of the sample is appropriate, a determination as to whether the blood sample is hemolyzed is made (S720). If the blood sample is hemolyzed, it is determined that a hemolysis-responsive test instrument is to be used.

If the turbidity is low (<TB1), it is determined that a non-hemolysis-responsive test instrument is to be used (S731).

If the blood sample is not hemolyzed, and the turbidity is low, a determination is made as to whether the total amount of proteins (TP) is appropriate for a non-hemolysis-responsive test instrument (whether condition 1 is satisfied, or >TP1) (S741).

If the turbidity is low (<TB1), and the total amount of proteins is appropriate (>TP1), the sample is diluted at a standard dilution ratio for a non-hemolysis-responsive test instrument (standard dilution ratio 1) (S751).

The blood sample diluted at the standard dilution ratio 1 is measured with a non-hemolysis-responsive test instrument (S761).

If the turbidity is not low (>TB1) (S731), and the turbidity is within a measurable range (>TB2) (S732), or if the total amount of proteins is also small (<TP1) (S741), the diluent is selected in accordance with the total amount of proteins. A determination is made as to whether the total amount of proteins (TP) is appropriate (>TP2) for a standard measurement performed by a hemolysis-responsive test instrument (S742).

If the total amount of proteins is not appropriate (<TP2) for the standard measurement performed by a hemolysis-responsive test instrument, the blood sample is diluted at a dilution ratio modified from the standard dilution ratio (S753).

The sample diluted at the standard dilution ratio 2 (S752) or the sample diluted at the modified dilution ratio (S753) is measured with a hemolysis-responsive test instrument (S762).

If the turbidity is excessively high (>TB2), it is determined that the measurement cannot be carried out even by a hemolysis-responsive test instrument (S732), and a result indicating that the measurement is impossible is returned (S763).

In the above embodiment, the measurement of the turbidity is described as two steps (S731 and S732); however, the measurement is not limited to this. For example, the measurement of the turbidity may be performed in a single turbidity measurement step.

### <Facility for Blood Test>

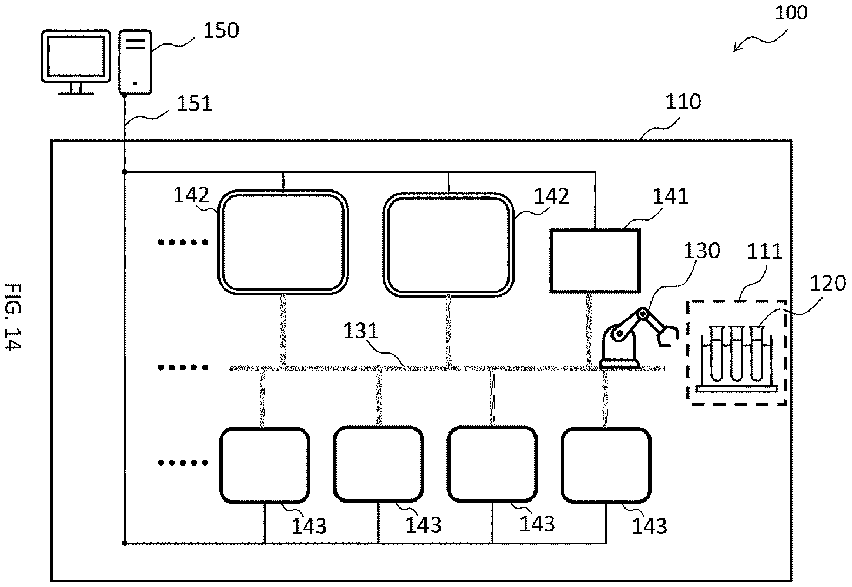

The present disclosure provides a facility for implementing the methods of the present disclosure. FIG. 14 schematically illustrates a configuration of a facility 100 for a blood test, according to an embodiment.

The blood test facility 100 includes a test room 110, which houses various instruments, 141, 142, and 143. The test room 110 is provided as a closed space so as to prevent the entry of contaminants and control the internal environments, such as temperature and humidity, to a certain level. A hemolysis determination instrument 141, a hemolysis-responsive test instrument 142, and a non-hemolysis-responsive test instrument 143 are placed in the test room 110. The test room 110 includes an introduction unit 111 for introducing a provided blood sample 120. The test room 110 includes a robot (transfer device or transfer system) 130 for transferring the blood sample 120 between the hemolysis determination instrument 141, the hemolysis-responsive test instrument 142, and the non-hemolysis-responsive test instrument 143. The robot 130 can move along a robot transfer line 131. The blood test facility 100 further includes a computer system 150 or is connected thereto. The computer system 150 is electrically or communicably connected to the various devices 141, 142, 143, the robot 130, the introduction unit 111, and the like, with a wire 151 or wirelessly (not illustrated).

When the blood sample 120 is provided from outside, the computer system 150 sends an instruction to the robot 130. The robot 130 moves to the introduction unit 111 and examines the attributes of the provided blood sample 120. For example, a measurement may be performed to determine whether the amount of the blood sample provided is not less than a predetermined amount. The determination of the amount of the blood sample may be carried out, for example, by determining, optically or with a camera, the liquid level of the sample present in the provided container or by measuring a weight of the sample. The robot 130 selects and acquires an appropriate blood sample 120, transfers the blood sample 120 to the hemolysis determination instrument 141, and introduces the blood sample 120 into the instrument.

The hemolysis determination instrument 141 performs a predetermined measurement on the introduced blood sample 120 and makes a determination regarding hemolysis. The hemolysis determination instrument 141 may measure an amount of proteins (total amount of proteins) present in the blood sample 120. By using the measurement results, the hemolysis determination instrument 141 determines whether the blood sample 120 is to be measured by the non-hemolysis-responsive test instrument 143 or determines which of the hemolysis-responsive test instrument 142 and the non-hemolysis-responsive test instrument 143 is to be used to measure the blood sample 120. The hemolysis determination instrument 141 sends the result to the computer system 150. Alternatively, the hemolysis determination instrument 141 may send the measurement result to the computer system 150. The computer system 150 can perform a similar determination.

If a determination that the measurement should be performed by the non-hemolysis-responsive test instrument 143 is made based on the result obtained in the hemolysis determination instrument 141, the computer system 150 instructs the robot 130 to transfer the blood sample 120 to the non-hemolysis-responsive test instrument 143. In response to the instruction, the robot 130 transfers the blood sample 120 from the hemolysis determination instrument 141 to the non-hemolysis-responsive test instrument 143 and introduces the blood sample 120 into the instrument.

The non-hemolysis-responsive test instrument 143 performs a predetermined measurement on the introduced blood sample 120. The non-hemolysis-responsive test instrument 143 sends the results obtained in the measurement to the computer system 150.

If a determination is made, based on the results obtained in the hemolysis determination instrument 141, that the measurement cannot be performed by the non-hemolysis-responsive test instrument 143 and, therefore, should be performed by the hemolysis-responsive test instrument 142, the computer system 150 instructs the robot 130 to transfer the blood sample 120 to the hemolysis-responsive test instrument 142. In response to the instruction, the robot 130 transfers the blood sample 120 from the hemolysis determination instrument 141 to the non-hemolysis-responsive test instrument 142 and introduces the blood sample 120 into the instrument.

The hemolysis-responsive test instrument 142 performs a predetermined measurement on the introduced blood sample 120. The hemolysis-responsive test instrument 142 sends the results obtained in the measurement to the computer system 150.

The computer system 150 receives the measurement results, performs a necessary calculation process, and sends the process results to a predetermined receiver.

When notified that the measurement has been completed, the computer system 150 receives the remainder of the blood sample 120 for the robot 130 and transfers the remainder to a disposal device (not illustrated). The facility 100 may include a storage apparatus or a storage container. The introduced sample, the remainder of the sample, the blood sample being in the process of a measurement, a sample that needs to be stored, and the like can be temporarily stored therein.

FIG. 14 illustrates one mobile robot for transferring the blood, and this is non-limiting. In some embodiments, the blood test facility may include a plurality of transfer robots. The transfer robots may operate in cooperation. In some embodiments, the blood test facility may have a transfer robot secured in the facility. The robot need not be a typical humanoid arm robot. The robot is capable of transferring the blood sample to a required test instrument or location at a required time.

While the transferring is performed by the robot in the above Example, the transferring may be performed, in some embodiments, by a human, such as an operator. In some embodiments, the transferring may be performed cooperatively by a robot and a human.

### <Illustrative Discussion>

In the case of hemolysis-responsive test instruments, such as an HPLC, measuring a large number of samples generally takes a long performance measurement time. In addition, the starting of the operation, which includes the determination of conditions, takes time and labor. If the instrument enters a downtime as a result of a failure or the like, restarting the operation takes time and labor. On the other hand, in the case of non-hemolysis-responsive test instruments that use or do not use an enzymatic method or the like, such as absorbance spectrometers, clinical test instruments, and automated analyzers, measuring a large number of samples generally takes a short performance measurement time. In addition, the starting of the operation and maintenance are relatively easy or can be accomplished in a short time.

A non-limiting comparative example will now be described. One HPLC needs a preparation time of seven hours per day and, for example, can measure three samples per hour. Specifically, in one example, one HPLC has a measurement efficiency of 30 samples per day. On the other hand, in one example, one absorbance spectrometer has 60 cells, can allow the exchange of samples every 12 seconds, and can carry nearly 100 analytes. In the case of absorbance spectrometers, calibration takes approximately 10 minutes, and using, for example, an automated introduction apparatus makes sequential exchange of samples possible. 300 or more samples per hour, for example, can be measured. Specifically, in one example, one absorbance spectrometer has a measurement efficiency of 7200 samples per day.

Accordingly, in instances where non-hemolysis-responsive test instruments suffice, the measurement should be performed by a non-hemolysis-responsive test instrument, and in instances where non-hemolysis-responsive test instruments do not suffice, the measurement should be performed by a hemolysis-responsive test instrument. Consequently, it is possible to very efficiently return desired test results regarding a large number of samples that are received.

The present disclosure includes the following embodiments.

A001 A method for testing a blood sample, the method comprising:
providing subject blood;
determining whether the subject blood is hemolyzed; and
if it is determined that the subject blood is hemolyzed, testing the subject blood with a test instrument that is responsive to hemolysis.

A001b A method for testing a blood sample, the method comprising:
determining whether subject blood is hemolyzed; and
if it is determined that the subject blood is hemolyzed, testing the subject blood with a test instrument that is responsive to hemolysis.

A011a A method for testing a blood sample, the method comprising:
providing subject blood;
providing a hemolysis-responsive test instrument and a non-hemolysis-responsive test instrument;
determining whether the subject blood is hemolyzed; and
if it is determined that the subject blood is not hemolyzed, testing the subject blood with the non-hemolysis-responsive test instrument, or if it is determined that the subject blood is hemolyzed, testing the subject blood with the hemolysis-responsive test instrument.

A011b A method for testing a blood sample, the method comprising:
providing subject blood;
providing a hemolysis-responsive test instrument and a non-hemolysis-responsive test instrument;
determining whether the subject blood is hemolyzed; and
if it is determined that the subject blood is hemolyzed, testing the subject blood with the hemolysis-responsive test instrument.

A011c A method for testing a blood sample, the method comprising:
providing subject blood;
providing a hemolysis-responsive test instrument and a non-hemolysis-responsive test instrument;
determining whether the subject blood is hemolyzed; and
if it is determined that the subject blood is not hemolyzed, testing the subject blood with the non-hemolysis-responsive test instrument.

A021 The method according to any one of embodiments A001 to A01 1c or any embodiment, wherein the hemolysis-responsive test instrument and the non-hemolysis-responsive test instrument are each a diabetes test instrument.

A022 The method according to any one of embodiments A001 to A021 or any embodiment, wherein the hemolysis-responsive test instrument is a high-performance liquid chromatography (HPLC) instrument, and the non-hemolysis-responsive test instrument is an absorbance spectrometer (that uses an enzymatic method).

A031 The method according to any one of embodiments A001 to A022 or any embodiment, wherein the determining whether the subject blood is hemolyzed comprises:
performing an absorbance measurement on the subject blood; and
determining, based on a difference in an absorbance between the subject blood and a control sample, whether the subject blood is hemolyzed.

A041 The method according to any one of embodiments A001 to A031 or any embodiment, wherein the testing the subject blood comprises measuring glycated albumin present in the subject blood.

A051 The method according to any one of embodiments A001 to A041 or any embodiment, further comprising measuring a protein present in the subject blood.

A052 The method according to embodiment A051, further comprising adjusting, based on a result of the measured protein, one or both of a measurement condition for a test that uses the hemolysis-responsive test instrument and a measurement condition for a test that uses the non-hemolysis-responsive test instrument.

A053 The method according to embodiment A051, further comprising:
measuring a total amount of proteins present in the subject blood; and
adjusting, based on the measured total amount of proteins, the condition for the test that uses the hemolysis-responsive test instrument.

A054 The method according to embodiment A053, wherein the condition for the test that uses the hemolysis-responsive test instrument includes a dilution ratio for diluting the subject blood for an HPLC method.

A061 The method according to any one of embodiments A001 to A054 or any embodiment, further comprising measuring an amount of the subject blood sample.

A062 The method according to embodiment A061, wherein if the amount of the subject blood sample is less than a predetermined amount, the method comprises testing the subject blood with the hemolysis-responsive test instrument.

A063 The method according to embodiment A061, wherein if the amount of the subject blood sample is less than a predetermined amount, the method comprises instructing a subject to have blood drawn again, notifying the subject that there is a possibility that the testing does not provide correct results, and/or providing an instruction or a notification, the instruction and the notification requiring the condition for the test to be adjusted.

A071 A method for testing a blood sample, the method comprising:
(a) providing subject blood;
(b) providing a hemolysis-responsive test instrument and a non-hemolysis-responsive test instrument;
(c) determining whether an amount of the subject blood sample is not less than a specified amount;
(d) determining whether the subject blood is hemolyzed;
(e) measuring a total amount of proteins present in the subject blood;
(f) if the amount of the subject blood sample is not less than the specified amount, and the subject blood sample is not hemolyzed, and when the total amount of proteins present in the subject blood is equal to or greater than a first specified value, determining that the subject blood is to be tested with the non-hemolysis-responsive test instrument;
(g) after (f), testing the subject blood with the non-hemolysis-responsive test instrument;
(h) if the amount of the subject blood sample is less than the specified amount, if the subject blood sample is hemolyzed, or if the total amount of proteins present in the subject blood is less than the first specified value, determining that the subject blood is to be tested with the hemolysis-responsive test instrument;
(i) if it is determined that the subject blood is to be tested with the hemolysis-responsive test instrument, and when the total amount of proteins present in the subject blood is equal to or greater than a second specified value, which is less than the first specified value, diluting the subject blood at a first standard dilution ratio;
(j) if it is determined that the subject blood is to be tested with the hemolysis-responsive test instrument, and when the total amount of proteins present in the subject blood is less than the first specified value and is equal to or greater than the second specified value that is less than the first specified value, diluting the subject blood at a second standard dilution ratio;
(k) if it is determined that the subject blood is to be tested with the hemolysis-responsive test instrument, and when the total amount of proteins present in the subject blood is less than the second specified value, diluting the subject blood at a modified dilution ratio that is less than the second standard dilution ratio; and
(l) after (j) or (k), testing the subject blood with the hemolysis-responsive test instrument.

A081 A method for testing a blood sample, the method comprising:
providing subject blood; and
and measuring an amount of the provided blood sample.

A082 The method according to embodiment A081, comprising, if it is determined that the subject blood is hemolyzed, testing the subject blood with a test instrument that is responsive to hemolysis.

A091 The method according to any one of embodiments A001 to A082 or any embodiment, further comprising measuring a turbidity of the subject blood.

A092 The method according to embodiment A091 or any embodiment, further comprising determining, based on the measured turbidity, that the testing is to be performed by the hemolysis-responsive test instrument, determining that the testing is to be performed by the non-hemolysis-responsive test instrument, or determining that a measurement is not to be performed.

A101 A method for testing a blood sample, the method comprising:
(a) providing subject blood;
(b) providing a hemolysis-responsive test instrument and a non-hemolysis-responsive test instrument;
(c) determining whether an amount of the subject blood sample is not less than a specified amount;
(d) determining whether the subject blood is hemolyzed;
(e) measuring a turbidity of the subject blood;
(f) measuring a total amount of proteins present in the subject blood;
(g) if the amount of the subject blood sample is not less than the specified amount, and the subject blood sample is not hemolyzed, and when the turbidity of the subject blood is less than a first specified value of the turbidity, and the total amount of proteins present in the subject blood is equal to or greater than a first specified value of the total amount of proteins, determining that the subject blood is to be tested with the non-hemolysis-responsive test instrument;
(h) after (g), testing the subject blood with the non-hemolysis-responsive test instrument;
(i) if the amount of the subject blood sample is less than the specified amount, if the subject blood sample is hemolyzed, if the turbidity of the subject blood sample, the turbidity of the subject blood is greater than the first specified value of the turbidity and less than a second specified value of the turbidity, or if the total amount of proteins present in the subject blood is less than the first specified value, determining that the subject blood is to be tested with the hemolysis-responsive test instrument;
(j) if it is determined that the subject blood is to be tested with the hemolysis-responsive test instrument, and when the total amount of proteins present in the subject blood is equal to or greater than a second specified value, which is less than the first specified value, diluting the subject blood at a first standard dilution ratio;
(k) if it is determined that the subject blood is to be tested with the hemolysis-responsive test instrument, and when the total amount of proteins present in the subject blood is less than the first specified value and is equal to or greater than the second specified value that is less than the first specified value, diluting the subject blood at a second standard dilution ratio;
(l) if it is determined that the subject blood is to be tested with the hemolysis-responsive test instrument, and when the total amount of proteins present in the subject blood is less than the second specified value, diluting the subject blood at a modified dilution ratio that is less than the second standard dilution ratio;
(m) after (k) or (l), testing the subject blood with the hemolysis-responsive test instrument; and
(n) if the turbidity of the subject blood sample, the turbidity of the subject blood is greater than the second specified value of the turbidity, determining that a measurement is not to be performed.

B001 A facility for implementing the method for testing a blood sample according to any one of embodiments A001 to A101 or any embodiment.

B011 A facility for implementing the method for testing a blood sample according to any one of embodiments A001 to A101 or any embodiment, the facility comprising:
a hemolysis-responsive test instrument;
a non-hemolysis-responsive test instrument; and
a computer system configured to be communicably connected to the hemolysis-responsive test instrument and the non-hemolysis-responsive test instrument.

B021 The facility for implementing the method for testing a blood sample according to embodiment B011 or any embodiment, wherein the hemolysis-responsive test instrument is configured to determine whether the subject blood is hemolyzed.

B022 The facility for implementing the method for testing a blood sample according to embodiment B011, B021, or any embodiment, further comprising a hemolysis determination instrument.

B023 The facility according to any one of embodiments B001 to B022 or any embodiment, further comprising an instrument that measures or determines an amount of the blood sample provided.

B024 The facility for implementing the method for testing a blood sample according to embodiment B011 or any embodiment, wherein the hemolysis-responsive test instrument is configured to measure or determine a turbidity of the subject blood.

B025 The facility according to any one of embodiments B001 to B024 or any embodiment, further comprising an instrument that measures or determines a turbidity of the blood sample provided.

B031 The facility for implementing the method for testing a blood sample according to any one of embodiments B001 to B025 or any embodiment, further comprising a robot that is configured to transfer the blood sample between the instruments and is communicable with the computer system.

B041 The facility for implementing the method for testing a blood sample according to embodiment B03 or any embodiment 1, wherein the computer system is configured to
cause the robot to transfer the provided blood sample to the hemolysis-responsive test instrument or the hemolysis determination instrument;
instruct the hemolysis-responsive test instrument or the hemolysis determination instrument to make a determination regarding hemolysis of the blood sample;
receive a result of the determination regarding hemolysis, and select which of the hemolysis-responsive test instrument and the non-hemolysis-responsive test instrument is to be used to test the hemolyzed sample;
instruct the robot to transfer the blood sample to the selected test instrument;
instruct the selected test instrument to perform a test on the transferred blood sample; and
receive a result of the test of the blood sample and output the result of the test.

B042 The facility for implementing the method for testing a blood sample according to embodiment B031, wherein the computer system is configured to
instruct the facility to transfer the provided blood sample to the hemolysis-responsive test instrument or the hemolysis determination instrument;
instruct the hemolysis-responsive test instrument or the hemolysis determination instrument to make a determination regarding hemolysis of the blood sample;
receive a result of the determination regarding hemolysis, and select which of the hemolysis-responsive test instrument and the non-hemolysis-responsive test instrument is to be used to test the hemolyzed sample;
instruct the facility to transfer the blood sample to the selected test instrument;
instruct the selected test instrument to perform a test on the transferred blood sample; and
receive a result of the test of the blood sample and output the result of the test.

D001 A computer program for implementing the method according to any one of embodiments A001 to A101 or any one embodiment.

D101 A program for performing an operation of the computer system according to embodiment B041, B042, or any embodiment.

E001 A storage medium for storing the program according to embodiment D001, D101, or any embodiment.

While several embodiments and examples of the present disclosure have been described above, these embodiments and examples illustratively describe the present disclosure. For example, the above embodiments are those described in detail so that the present disclosure can be clearly described, and, if necessary, additional modifications may be made to dimensions, configurations, materials, and circuits. Note that the scope of the present disclosure encompasses embodiments in which one or more of the above-described features of the present disclosure are desirably combined. It is intended that the appended claims cover numerous modifications to the embodiments within the technical spirit of the present disclosure. Accordingly, it is to be understood that the embodiments and examples disclosed herein have been presented by way of illustration and should not be considered as limiting the scope of the present disclosure.

## Claims

1. A method for testing a blood sample, the method comprising:
providing subject blood;
providing a hemolysis-responsive test instrument and a non-hemolysis-responsive test instrument;
determining whether the subject blood is hemolyzed; and
if it is determined that the subject blood is not hemolyzed, testing the subject blood with the non-hemolysis-responsive test instrument, or if it is determined that the subject blood is hemolyzed, testing the subject blood with the hemolysis-responsive test instrument.

2. The method according to Claim 1, wherein the hemolysis-responsive test instrument and the non-hemolysis-responsive test instrument are each a diabetes test instrument.

3. The method according to Claim 1, wherein the hemolysis-responsive test instrument is a high-performance liquid chromatography (HPLC) instrument, and the non-hemolysis-responsive test instrument is an absorbance spectrometer.

4. The method according to Claim 1, wherein the determining whether the subject blood is hemolyzed comprises:
performing an absorbance measurement on the subject blood; and
determining, based on a difference in an absorbance between the subject blood and a control sample, whether the subject blood is hemolyzed.

5. The method according to Claim 1, wherein the testing the subject blood comprises measuring glycated albumin present in the subject blood.

6. The method according to Claim 1, further comprising:
measuring a protein present in the subject blood; and
adjusting, based on a result of the measured protein, one or both of a measurement condition for a test that uses the hemolysis-responsive test instrument and a measurement condition for a test that uses the non-hemolysis-responsive test instrument.

7. The method according to Claim 6, wherein the condition for the test that uses the hemolysis-responsive test instrument includes a dilution ratio for diluting the subject blood for an HPLC method.

8. The method according to Claim 1, further comprising measuring an amount of the subject blood sample.

9. The method according to Claim 8, wherein if the amount of the subject blood sample is less than a predetermined amount, the method comprises testing the subject blood with the hemolysis-responsive test instrument.

10. The method according to Claim 8, wherein if the amount of the subject blood sample is less than a predetermined amount, the method comprises instructing a subject to have blood drawn again, notifying the subject that there is a possibility that the testing does not provide correct results, and/or providing an instruction or a notification, the instruction and the notification requiring the condition for the test to be adjusted.

11. The method according to Claim 1, further comprising measuring a turbidity of the subject blood.

12. The method according to Claim 11, further comprising determining, based on the measured turbidity, that the testing is to be performed by the hemolysis-responsive test instrument, determining that the testing is to be performed by the non-hemolysis-responsive test instrument, or determining that a measurement is not to be performed.

13. A facility for implementing the method for testing a blood sample according to any one of Claims 1 to 12, the facility comprising:
a hemolysis-responsive test instrument;
a non-hemolysis-responsive test instrument; and
a computer system configured to be communicably connected to the hemolysis-responsive test instrument and the non-hemolysis-responsive test instrument.

14. The facility for implementing the method for testing a blood sample according to Claim 13, wherein the hemolysis-responsive test instrument is configured to determine whether the subject blood is hemolyzed.

15. The facility for implementing the method for testing a blood sample according to Claim 13, further comprising a hemolysis determination instrument.

16. The facility for implementing the method for testing a blood sample according to Claim 13, further comprising a robot that is configured to transfer the blood sample between the instruments and is communicable with the computer system.

17. The facility for implementing the method for testing a blood sample according to Claim 16, wherein the computer system is configured to
cause the robot to transfer the provided blood sample to the hemolysis-responsive test instrument or the hemolysis determination instrument;
instruct the hemolysis-responsive test instrument or the hemolysis determination instrument to make a determination regarding hemolysis of the blood sample;
receive a result of the determination regarding hemolysis, and select which of the hemolysis-responsive test instrument and the non-hemolysis-responsive test instrument is to be used to test the hemolyzed sample;
instruct the robot to transfer the blood sample to the selected test instrument;
instruct the selected test instrument to perform a test on the transferred blood sample; and
receive a result of the test of the blood sample and output the result of the test.

18. The facility for implementing the method for testing a blood sample according to Claim 16, wherein the computer system is configured to
instruct the facility to transfer the provided blood sample to the hemolysis-responsive test instrument or the hemolysis determination instrument;
instruct the hemolysis-responsive test instrument or the hemolysis determination instrument to make a determination regarding hemolysis of the blood sample;
receive a result of the determination regarding hemolysis, and select which of the hemolysis-responsive test instrument and the non-hemolysis-responsive test instrument is to be used to test the hemolyzed sample;
instruct the facility to transfer the blood sample to the selected test instrument;
instruct the selected test instrument to perform a test on the transferred blood sample; and
receive a result of the test of the blood sample and output the result of the test.

19. A program for performing an operation of the computer system according to Claim 17 or 18.

## Amended claims

### Amended claims under Art. 19.1 PCT

1. A method for testing a blood sample, the method comprising:
providing subject blood;
providing a hemolysis-responsive test instrument and a non-hemolysis-responsive test instrument;
determining whether the subject blood is hemolyzed; and
if it is determined that the subject blood is not hemolyzed, performing a test with the non-hemolysis-responsive test instrument, the test including a measurement of glycated albumin present in the subject blood, or if it is determined that the subject blood is hemolyzed, performing a test with the hemolysis-responsive test instrument, the test including a measurement of glycated albumin present in the subject blood.

2. The method according to Claim 1, wherein the hemolysis-responsive test instrument and the non-hemolysis-responsive test instrument are each a diabetes test instrument.

3. The method according to Claim 1, wherein the hemolysis-responsive test instrument is a high-performance liquid chromatography (HPLC) instrument, and the non-hemolysis-responsive test instrument is an absorbance spectrometer.

4. The method according to Claim 1, wherein the determining whether the subject blood is hemolyzed comprises:
performing an absorbance measurement on the subject blood; and
determining, based on a difference in an absorbance between the subject blood and a control sample, whether the subject blood is hemolyzed.

6. The method according to Claim 1, further comprising:
measuring a protein present in the subject blood; and
adjusting, based on a result of the measured protein, one or both of a measurement condition for a test that uses the hemolysis-responsive test instrument and a measurement condition for a test that uses the non-hemolysis-responsive test instrument.

7. The method according to Claim 6, wherein the condition for the test that uses the hemolysis-responsive test instrument includes a dilution ratio for diluting the subject blood for an HPLC method.

8. The method according to Claim 1, further comprising measuring an amount of the subject blood sample.

9. The method according to Claim 8, wherein if the amount of the subject blood sample is less than a predetermined amount, the method comprises testing the subject blood with the hemolysis-responsive test instrument.

10. The method according to Claim 8, wherein if the amount of the subject blood sample is less than a predetermined amount, the method comprises instructing a subject to have blood drawn again, notifying the subject that there is a possibility that the testing does not provide correct results, and/or providing an instruction or a notification, the instruction and the notification requiring the condition for the test to be adjusted.

11. The method according to Claim 1, further comprising measuring a turbidity of the subject blood.

12. The method according to Claim 11, further comprising determining, based on the measured turbidity, that the testing is to be performed by the hemolysis-responsive test instrument, determining that the testing is to be performed by the non-hemolysis-responsive test instrument, or determining that a measurement is not to be performed.

13. A facility for implementing the method for testing a blood sample according to any one of Claims 1 to 12, the facility comprising:
a hemolysis-responsive test instrument;
a non-hemolysis-responsive test instrument; and
a computer system configured to be communicably connected to the hemolysis-responsive test instrument and the non-hemolysis-responsive test instrument.

14. The facility for implementing the method for testing a blood sample according to Claim 13, wherein the hemolysis-responsive test instrument is configured to determine whether the subject blood is hemolyzed.

15. The facility for implementing the method for testing a blood sample according to Claim 13, further comprising a hemolysis determination instrument.

16. The facility for implementing the method for testing a blood sample according to Claim 13, further comprising a robot that is configured to transfer the blood sample between the instruments and is communicable with the computer system.

17. The facility for implementing the method for testing a blood sample according to Claim 16, wherein the computer system is configured to
cause the robot to transfer the provided blood sample to the hemolysis-responsive test instrument or the hemolysis determination instrument;
instruct the hemolysis-responsive test instrument or the hemolysis determination instrument to make a determination regarding hemolysis of the blood sample;
receive a result of the determination regarding hemolysis, and select which of the hemolysis-responsive test instrument and the non-hemolysis-responsive test instrument is to be used to test the hemolyzed sample;
instruct the robot to transfer the blood sample to the selected test instrument;
instruct the selected test instrument to perform a test on the transferred blood sample; and
receive a result of the test of the blood sample and output the result of the test.

18. The facility for implementing the method for testing a blood sample according to Claim 16, wherein the computer system is configured to
instruct the facility to transfer the provided blood sample to the hemolysis-responsive test instrument or the hemolysis determination instrument;
instruct the hemolysis-responsive test instrument or the hemolysis determination instrument to make a determination regarding hemolysis of the blood sample;
receive a result of the determination regarding hemolysis, and select which of the hemolysis-responsive test instrument and the non-hemolysis-responsive test instrument is to be used to test the hemolyzed sample;
instruct the facility to transfer the blood sample to the selected test instrument;
instruct the selected test instrument to perform a test on the transferred blood sample; and
receive a result of the test of the blood sample and output the result of the test.

19. A program for performing an operation of the computer system according to Claim 17 or 18.

Statement under Art. 19.1 PCT
The applicant has amended Claim 1 to incorporate the feature of Claim 5 as originally filed or a feature substantially equivalent thereto.

Accordingly, Claim 5 has been canceled.
